# EUROPÄISCHE PATENTSCHRIFT

(11) **EP 1 205 472 B1**
(45) Veröffentlichungstag und Bekanntmachung des Hinweises auf die Patenterteilung: **26.01.2005**
(21) Anmeldenummer: 01125353.1
(22) Anmeldetag: 29.10.2001
(51) Int. Cl.: C07D 277/78, C07D 417/14

(54) **Verfahren zur Herstellung von Dithiazolyldisulfiden**
Process for the preparation of dithiazolyldisulfides
Procédé de préparation de disulfures de thiazole

(30) Priorität: 08.11.2000 DE 10055219
(43) Veröffentlichungstag der Anmeldung: 15.05.2002
(73) Patentinhaber: BAYER AG, 51368 Leverkusen (DE)
(72) Erfinder: Wolber, Wolfgang, 50126 Bergheim (DE); Oberthür, Markus, Dr., 41540 Dormagen (DE)

(56) Entgegenhaltungen:
- EP-A- 0 194 571
- DE-A- 2 349 314
- US-A- 4 337 344
- US-A- 4 463 178
- REPKINA V.I.; PTITSYNA V.V.; LATYSHEVA L.M.: J. APPL. CHEM. USSR (ENGL. TRANSL.), Bd. 57, 1984, Seiten 1082-1084, XP001037091 -& REPKINA V.P., PTITSYNA V.V AND LATYSHEVA L.M.: "oxidation of 2-mercaptobenzothiazole by hydroogen peroxyde in dilute aqueous solutions" J. APPL. CHEM. USSR, Bd. 57, 1984, Seite 180-181 XP001038637
- SETO S. ET AL.: BULL. CHEM.SOC. JAPAN, Bd. 35, 1962, Seiten 1998-2002, XP001038439

## Beschreibung

Die Erfindung betrifft ein Verfahren zur Herstellung von Dithiazolyl-(2,2')-disulfiden durch Oxidation von 2-Mercaptothiazolen mit peroxidischen Verbindungen in wässriger Suspension in einem bestimmten pH-Bereich.

Bei der technischen Herstellung von Dibenzothiazyldisulfiden durch Oxidation von 2-Mercaptobenzthiazolen wurden bereits die verschiedensten Oxidationsmittel verwendet (Ullmanns Encyclopedia of Industrial Chemistry, 5^{th} ed. Vol. A-26, p. 773-8, VCH, Weinheim, Basel, Cambridge, New York, Tokyo, 1995). So ist die Oxidation mit Natriumchlorat und Natriumnitritlösung in salzsaurem Medium bei 30°C Stand der Technik. Dieses Verfahren ist jedoch mit einer Reihe von Nachteilen behaftet. Der Verbrauch an Mineralsäure ist sehr hoch (3 Mole HCl je Mol 2-Mercaptobenzthiazol) und es entstehen große Mengen an Beiprodukten. Weiterhin ist es bekannt, die Oxidation des 2-Mercaptobenzthiazolen mittels salpetriger Säure durchzuführen. Nach dem Verfahren der US-PS 19 08 935 wird 2-Mercaptobenzthiazol in Wasser suspendiert, ein wasserlösliches Nitrit zugefügt und Sauerstoff oder ein sauerstoffhaltiges Gas, wie Luft, durch das Reaktionsgemisch geleitet. Gleichzeitig wird eine Mineralsäure, welche aus dem Nitrit salpetrige Säure freisetzt, zugefügt. Beim Verfahren gemäß US-PS 21 19 131 und US-PS 3 062 825 werden stöchiometrische Mengen Nitrit als alleiniges Oxidationsmittel eingesetzt. Hierdurch wird eine raschere und vollständigere Umsetzung erzielt. Diese Oxidationsverfahren sind insofern ebenfalls nachteilig, weil auch hier der Verbrauch an Mineralsäure sehr hoch ist und Salze sowie Stickoxide in großen Mengen als Nebenprodukte anfallen.

Auch Chlor wurde bereits als Oxidationsmittel eingesetzt (Kirk-Othmer, Encyclopedia of Polymer Science and Technology (1970), Vol. 12, S. 262). Hierbei handelt es sich jedoch um eine komplizierte Reaktion mit kritischen Reaktionsbedingungen bei der häufig große Mengen an überoxidierten Nebenprodukten anfallen. Nach DE-A 23 09 584 werden zwecks Erhöhung der Produktausbeute und Verminderung der für eine ausreichende Oxidation benötigten Menge an überschüssigem Chlor unter der Oberfläche der Flüssigkeit unter starkem Rühren kontinuierlich getrennte Ströme aus einer wäßrigen Lösung eines Alkalimetallsalzes von Mercaptobenzthiazol, einer wäßrigen Lösung eines Alkalimetallhydroxids und gasförmiges Chlor bei 20 bis 75° miteinander umgesetzt, wobei der pH-Wert und das Redoxpotential der wäßrigen Mischung durch Regelung des Zuflusses der wäßrigen Hydroxidlösung und des gasförmigen Chlors bei pH 7 bis 10 und einem Redoxpotential von -150 bis 250 mV gehalten werden. Auch dieses Verfahren bedarf einer sehr sorgfältigen Steuerung um die Weiteroxidation des Dibenzothiazyldisulfids zu Benzothiazyl-2-sulfinat und -sulfonat zu verhindern. Nachteilig ist das Verfahren auch deshalb, weil große Mengen Alkalihydroxid verbraucht und große Mengen Kochsalz als Beiprodukt entstehen.

Hydroperoxide, wie Wasserstoffperoxid, Alkylhydroperoxide und Aralkylhydroperoxide, wurden ebenfalls bereits als Oxidationsmittel bei der Herstellung von Dibenzothiazyldisulfid eingesetzt (siehe z.B. DE-A 23 49 314). Hierbei wird jedoch ausdrücklich die Verwendung eines niederen aliphatischen Alkohols als Lösungsmittel gefordert. Die Verwendung von organischen Lösungsmitteln ist jedoch nachteilig für ein technisches Verfahren, da von ihnen sowohl eine Umweltgefährdung, als auch, wegen ihrer leichten Entzündbarkeit, eine stete Brandgefährdung ausgehen kann. Weiterhin müssen organische Lösungsmittel nach ihrer Verwendung aufwendig rezykliert, gereinigt und entsorgt werden.

Die Oxidation von heterocyclischen Thiolen zu Disulfiden mit Hilfe von Wasserstoffperoxid oder organischen Persäuren in Wasser oder organischen Lösungsmitteln oder in Gemischen daraus ist in EP-A 194 571 A1 beschrieben. Ein bestimmter pH, der bei diesen Reaktionen zwingend eingehalten werden soll, wird darin jedoch nicht offenbart. In den Beispielen findet sich nur eine einzige Reaktion in wässrigem Milieu, wobei jedoch das Thiol in großer Verdünnung (2 %ige Lösung) und in homogen gelöster Form mit Wasserstoffperoxid umgesetzt wird. Auf diese, hierin beschriebene Weise wird sehr viel Wasser bei der Reaktion benötigt und eine entsprechend große Menge an Abwasser erzeugt, die kostenintensiv entsorgt werden muss. Eine Möglichkeit, heterogen in konzentrierter Suspension zu arbeiten und dadurch Lösungsmittel bzw. Abwasser zu sparen, wird nicht offenbart.

Diese Möglichkeit der Umsetzung von 2-Mercaptothiazolen mit Hydroperoxiden, speziell mit Wasserstoffperoxid, in konzentrierter wässriger Suspension ist in US-A 4 463 178 beschrieben. Gemäß dieser Veröffentlichung wird jedoch als Lösungsvermittler für das ansonsten in Wasser unlösliche 2-Mercaptothiazol die Verwendung einer wässrigen Aminlösung, wie z.B. Ammoniak- oder Alkylaminlösung, explizit gefordert. Es wird nicht offenbart, dass die Oxidationsreaktion problemlos und mit quantitativen Ausbeuten auch ohne jedes lösungsvermittelnde Hilfsagens in reinem Wasser ablaufen kann.

EP-A 008 548 beschreibt die Verwendung von Wasserstoffperoxid als Oxidationsmittel in Kombination mit Ethylendiamintetraessigsäure oder ihren Salzen. Die Reaktionszeiten müssen hierbei sehr lang sein, zum Teil über 24 Stunden, um vollständigen Umsatz zu erreichen. Dies ist für einen großtechnischen Prozeß ein großer Nachteil.

Allen obengenannten Oxidationsverfahren gemeinsam ist der Nachteil, daß vergleichsweise teure Oxidationsmittel sowie Säuren, Basen, Lösungsmittel oder andere Hilfsstoffe benötigt werden, wobei zum Teil auch nichtverwertbare Bei- oder Nebenprodukte anfallen.

Zu erwähnen ist auch noch ein Verfahren der elektrolytischen Oxidation von 2-Mercaptobenzothiazol zu Dibenzothiazyldisulfid (siehe DE-A 27 43 629). Dieses Verfahren ist technisch aufwendig und daher weniger wirtschaftlich.

Darüber hinaus sind auch aus
Repkina V.I.; Ptitsyna V.V.; Latysheva L.M. J. Appl. Chem. USSR (Engl. Transl.), Bd. 57, 1984 Seiten 1082-1084, XP001037091 & Repkina V.P, Ptitsyna V.V. and Latysheva L.M., J. Appl. Chem. USSR, Bd. 57, 1984, Seiten 180-181 XP001038637;
Seto S. et. al., Bull Chem. Soc. Japan, Bd. 35, 1962, Seiten 1998-2002, XP001038439;
US-A-4 337 344 (Alicot Michel J C et al.) 29. Juni 1982;
US-A-4 463 178 (Tazuma James J) 31. Juli 1984;
DE 23 49 314 A (Goodyear Tire & Rubber) 9. Mai 1974 und
EP-A 0 194 571 (Hoechst AG) 17. September 1986

Verfahren zur Herstellung von Dithiazoyl-(2,2')-disulfiden durch Oxidation von entsprechenden Mercaptothiazolen mit peroxidischen Verbindungen in wässriger Suspension bekannt. Allerdings werden diese Verfahren unter den verschiedensten Reaktionsbedingurigen durchgeführt, nicht jedoch in einer wässrigen Suspension und nur im pH-Bereich von 6,5 bis 8, wie im nachstehend beschriebenen erfindungsgemäßen Verfahren es der Fall ist. Daher sind alle die genannten Verfahren in den aufgeführten Dokumenten ebenfalls mit Nachteilen behaftet, wie sie bereits beschrieben wurden.

Es besteht daher nach wie vor das Bedürfnis zur Schaffung eines verbesserten Verfahrens für die Oxidation von 2-Mercaptothiazolen mittels peroxidischen Verbindungen.

Gegenstand der vorliegenden Erfindung ist daher ein Verfahren zur Herstellung von Dithiazolyl-(2,2')-disulfiden der allgemeinen Formel 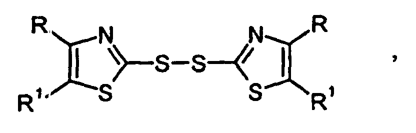 wobei
- R und R¹: gleich oder verschieden sein können und jeweils für Wasserstoff, Halogen, Nitro, Hydroxyl oder gegebenenfalls substituiertes C₁-C₁₂-Alkyl oder -Alkoxyl oder C₆-C₁₂-Cycloalkyl oder -Aryl oder C₁-C₁₂-Heteroaryl stehen oder gemeinsam den Rest 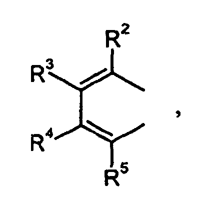
wobei
- R² bis R⁵: gleich oder verschieden sein können und jeweils für Wasserstoff, Halogen, Nitro, Hydroxyl oder gegebenenfalls substituiertes C₁-C₁₂-Alkyl oder -Alkoxyl oder C₆-C₁₂-Cycloalkyl oder -Aryl oder C₁-C₁₂-Heteroaryl stehen,
bilden, durch Oxidation eines entsprechenden substituierten 2-Mercaptothiazols mit peroxidischen Verbindungen, dadurch gekennzeichnet, daß die Oxidation in wässriger Suspension bei einem pH-Wert im Bereich von 6,5 bis 8,0, bevorzugt 6,8 bis 7,5, durchgeführt wird.

Als Halogenreste der obigen Formel kommen Fluor, Chlor, Brom oder Jod, bevorzugt Chlor oder Brom in Betracht.

Unter C₁-C₁₂-Alkyl werden sämtliche dem Fachmann bekannte lineare oder verzweigte Alkylreste mit 1 bis 12 C-Atomen verstanden, wie Methyl, Ethyl, n-Propyl, i-Propyl, n-Butyl, i-Butyl, t-Butyl, n-Pentyl, i-Pentyl, neo-Pentyl und Hexyl, die ihrerseits wiederum substituiert sein können. Als Substituenten kommen hierbei Halogen, Nitro, Hydroxyl, oder auch C₁-C₁₂-Alkyl oder -Alkoxy, sowie C₆-C₁₂-Cycloalkyl oder -Aryl in Frage, wie Benzoyl, Trimethylphenyl, Ethylphenyl, Chlormethyl, Chlorethyl und Nitromethyl.

Unter C₁-C₁₂-Alkoxyl werden sämtliche dem Fachmann bekannte lineare oder verzweigte Alkoxylreste mit 1 bis 12 C-Atomen verstanden, wie Methoxy, Ethoxy, n-Propoxy, i-Propoxy, n-Butoxy, i-Butoxy, t-Butoxy, n-Pentoxy, i-Pentoxy, neo-Pentoxy und Hexoxy, die ihrerseits wiederum substituiert sein können. Als Substituenten kommen hierbei Halogen, Nitro, Hydroxyl, oder auch C₁-C₁₂-Alkyl oder Alkoxyl, sowie C₆-C₁₂-Cycloalkyl oder -Aryl in Frage.

Unter C₆-C₁₂-Cycloalkyl werden sämtliche dem Fachmann bekannte ein- oder mehrkernige Cycloalkylreste mit 6 bis 12 C-Atomen verstanden, wie Cyclohexyl, Cycloheptyl, Cyclooctyl und Cyclononyl, die ihrerseits wiederum substituiert sein können. Als Substituenten kommen hierbei Halogen, Nitro, Hydroxyl, oder auch C₁-C₁₂-Alkyl oder -Alkoxyl, sowie C₆-C₁₂-Cycloalkyl oder -Aryl in Frage, wie Methylcyclohexyl, Chlorcyclohexyl und Nitrocyclohexyl.

Unter C₆-C₁₂-Aryl werden sämtliche dem Fachmann bekannte ein- oder mehrkernige Arylreste mit 6 bis 12 C-Atomen verstanden, wie Phenyl, Naphthyl, die ihrerseits wiederum substituiert sein können. Als Substituenten kommen hierbei Halogen, Nitro, Hydroxyl, oder auch C₁-C₁₂-Alkyl oder -Alkoxyl, sowie C₆-C₁₂-Cycloalkyl oder -Aryl in Frage, wie Bromphenyl, Chlorphenyl, Toluyl und Nitrophenyl.

Unter C₁-C₁₂-Heteroaryl werden sämtliche dem Fachmann bekannten ein- oder mehrkernigen Heteroarylreste verstanden, die neben 1 bis 12 C-Atomen noch ein oder mehrere Heteroatome, wie N, S, O und/oder P, im aromatischen Ringsystem tragen, wie Pyridinyl, Triazinyl, Furyl, Thienyl, Thiazolyl, Thiazinyl, Pyrrolyl, Chinolinyl, die ihrerseits wiederum durch die obengenannten Substituenten substituiert sein können.

Bevorzugt stehen die Reste R-R⁵ in der Formel für Wasserstoff, Methyl, Ethyl, Propyl, t-Butyl, Methoxy, Ethoxy, Cyclohexyl, Benzoyl, Methoxy, Ethoxy, Phenyl, Naphthyl, Chlorphenyl, Toluyl und Nitrophenyl.

Dithiazolyl-(2,2')-disulfide werden beispielsweise als Vulkanisiermittel für Gummi verwendet. Das erfindungsgemäße Verfahren ist insbesondere für die Herstellung von Dibenzothiazolyl-(2,2')-disulfid, dem wichtigsten Vertreter dieser Verbindungsklasse, bedeutsam. Es ist jedoch ebenso mit Erfolg bei der Herstellung weiterer Verbindungen dieses Typs geeignet. Für die bevorzugte Herstellung von Dibenzothiazolyl-(2,2')-disulfid (MBTS) wird 2-Mercaptobenzthiazol (MBT) als Ausgangsstoff eingesetzt. Beispiele anderer 2-Mercaptothiazole, welche sich als Ausgangsstoffe für die Herstellung weiterer Dithiazolyl-(2,2')-disulfide der allgemeinen Formel (I) eignen, sind u.a. die in DE-A 23 55 897 genannten Verbindungen, wie
2-Mercaptothiazol
2-Mercapto-4-methylthiazol
2-Mercapto-4-ethylthiazol
2-Mercapto-4n-propylthiazol
2-Mercapto-4n-butylthiazol
2-Mercapto-4,5-dimethylthiazol
2-Mercapto-4,5-di-n-butylthiazol
2-Mercapto-4-phenylthiazol
2-Mercapto-5-chlor-4-phenylthiazol
2-Mercapto-4-p-bromphenylthiazol
2-Mercapto-4-m-nitrophenylthiazol
2-Mercapto-4-m-chlorphenylthiazol
2-Mercapto-4-methylbenzothiazol
2-Mercapto-5-methylbenzothiazol
2-Mercapto-6-methylbenzothiazol
2-Mercapto-4,5-dimethylbenzothiazol
2-Mercapto-4-phenylbenzothiazol
2-Mercapto-4-methoxybenzothiazol
2-Mercapto-6-methoxybenzothiazol
2-Mercapto-5,6-dimethoxybenzothiazol
2-Mercapto-6-methoxy-4-nitrobenzothiazol
2-Mercapto-6-ethoxybenzothiazol
2-Mercapto-4-chlorbenzothiazol
2-Mercapto-5-chlorbenzothiazol
2-Mercapto-6-chlorbenzothiazol
2-Mercapto-7-chlorbenzothiazol
2-Mercapto-5-chlor-6-methoxybenzothiazol
2-Mercapto-5-chlor-4-nitrobenzothiazol
2-Mercapto-5-chlor-6-nitrobenzothiazol
2-Mercapto-4,5-dichlorbenzothiazol
2-Mercapto-4,7-dichlorbenzothiazol
2-Mercapto-5-nitrobenzothiazol
2-Mercapto-6-nitrobenzothiazol
2-Mercapto-4-phenylbenzothiazol
2-Mercapto-naphthothiazol
2-Mercapto-6-hydroxybenzothiazol.

Als Oxidationsmittel werden - wie erwähnt - peroxidischen Verbindungen, insbesondere Wasserstoffperoxid, Alkylhydroperoxide oder Aralkylhydroperoxide eingesetzt. Selbstverständlich können auch Gemische aus diesen eingesetzt werden. Als Alkylhydroperoxide und Aralkylhydroperoxide können sämtliche in DE-A-2 349 314 beschriebene Peroxide eingesetzt werden. Bevorzugt ist jedoch Wasserstoffperoxid. Im allgemeinen liegt beim erfindungsgemäßen Verfahren die eingesetzte Peroxidkonzentration im Bereich von 3 bis 50 Gew.-%. Aus ökonomischen Gründen werden vorzugsweise Peroxidkonzentrationen von 5 bis 35 Gew.-% angewendet, besonders bevorzugt von 10 bis 35 Gew.-%

Als Lösungsmittel für das erfindungsgemäße Verfahren dient Wasser. Dem Wasser können jedoch noch wassermischbare oxidationsstabile organische Lösungsmittel zugesetzt werden. Beispiele hierfür sind Alkohole und Ketone, Dimethylformamid und Aceton sowie deren Mischungen. Geeignete Alkohole sind beispielsweise aliphatische Alkohole mit 1 bis 10 Kohlenstoffatomen, insbesondere Methanol, Ethanol, Propanol, Isopropanol, n-Butanol, sec.-Butanol, tert.-Butanol, Pentanol, Hexanol, Heptanol und Octanol. Die Konzentration des Lösungsmittels im Wasser ist nicht kritisch. Im allgemeinen liegt die Lösungsmittelmenge im Bereich von 1 bis 10 Gew.-%, bezogen auf die Menge an Wasser. Größere Mengen Lösungsmittel sind aus ökonomischen Gründen zu vermeiden, da in diesen Fällen auch größere Mengen Lösungsmittel aufgearbeitet oder entsorgt werden müssen.

Selbstverständlich ist es auch möglich die Peroxide, insbesondere das Wasserstoffperoxid, in situ aus geeigneten Vorläufen herzustellen oder freizusetzen.

Der pH-Wert des erfindungsgemäßen Verfahrens muss vom Beginn der Reaktion bis zu ihrem Ende zwingend im angegebenen Bereich liegen. Wird dieser Bereich unterschritten, so sinken Ausbeute und Reinheit des Endproduktes. Wird der pH-Bereich überschritten, so sinken Ausbeute und Selektivität der Reaktion und es werden Nebenprodukte erzeugt, die ins Endprodukt wie auch ins Abwasser gelangen. Dadurch wird das erfindungsgemäße Produkt verunreinigt und das Abwasser mit organischem Material belastet.

Um pH-Schwankungen während der Reaktion vorzubeugen und die Reaktion exakt im angegebenen pH-Bereich zu halten, kann eine effiziente Mess- und Regelungstechnik verwendet werden, die beispielsweise aus einer in-situ pH-Messung und einem elektronisch gesteuerten Dosiersystem für die je nach Bedarf zuzufügende Säure bzw. Base besteht.

Geeignete Säuren und Basen sind dem Fachmann bekannt, ihre Auswahl ist nicht kritisch. Vorzugsweise verwendete Säuren sind beispielsweise Schwefel-, Salz- oder Phosphorsäure. Bevorzugte Basen sind z.B. wässrige Lösungen von Ammoniumhydroxid oder von basischen Metallhydroxiden der Gruppen 1 bis 13 des Periodensystems der Elemente, bevorzugt Natrium- oder Kaliumhydroxid.

Um auch kleinere pH-Schwankungen sehr schnell ausgleichen zu können, verwendet man bevorzugt ein Puffersystem, das dem erforderlichen pH-Bereich angepasst ist. Für diesen Zweck geeignete Puffer sind dem Fachmann bekannt und beispielsweise im "Römpp Chemie Lexikon", Thieme Verlag Stuttgart, 9. Aufl., Bd. 5, (1992) 3677 oder im "CRC Handbook of Chemistry and Physics" 79^{th} Ed. (1998) **8**-43, zu finden.

Beispielsweise können Puffer eingesetzt werden, die sich zusammensetzen aus einem Gemisch von Metallhydrogencarbonaten und Metallcarbonaten oder aus einem Gemisch von Metallhydrogenphosphat und Metallphosphat, wobei die Metalle den Gruppen 1 bis 13 des Periodensystems der chemischen Elemente entstammen, sowie beispielsweise aus einem Gemisch von stickstoffhaltigen Basen, wie Triethanolamin, Tris(hydroxymethyl)aminomethan oder Imidazol und ihren Ammoniumsalzen, die durch Reaktion mit einer Säure, z.B. Salzsäure oder Schwefelsäure, entstehen. Die Komponenten des Puffers, z.B. die erwähnten Metallsalze, können dabei einzeln oder im Gemisch miteinander eingesetzt werden.

Bevorzugt werden Puffer verwendet, die sich zusammensetzen aus einem Gemisch der Natrium- oder Kaliumsalze mit Anionen aus -hydrogencarbonat und -carbonat oder aus -dihydrogenphosphat, -hydrogenphosphat und -phosphat oder aus Tris(hydroxymethyl)aminomethan und seinen Ammoniumsalzen.

Der Puffer kann sowohl als alleinige pH-Steuerung, als auch bevorzugt in Kombination mit einem effizienten Regelungssystem, wie oben beschrieben, angewendet werden.

Die Menge des verwendete Puffers und die entsprechende Pufferkapazität richten sich nach der Stärke der zu erwartenden pH-Schwankung, die wiederum abhängig ist von den Reatkionsbedingungen, wie Temperatur und Dosiergeschwindigkeit und der Leistungsfähigkeit des verwendeten pH-Regelungssystems, und ist leicht durch entsprechende Vorversuche zu ermitteln.

Die Reaktionstemperatur beim erfindungsgemäßen Verfahren beträgt etwa 0 bis 150°C, vorzugsweise 20 bis 90°C und insbesondere 30 bis 70°C. Bei tieferen Temperaturen nimmt die Reaktionsgeschwindigkeit ab, bei höheren Temperaturen verringert sich die Selektivität der Reaktion.

Die Reaktionsdauer beträgt in der Regel 0,5 bis 10 Stunden unter den genannten Reaktionsbedingungen.

Die Durchführung des erfindungsgemäßen Verfahrens erfolgt in einfacher Weise z.B. dadurch, daß das 2-Mercaptothiazol in pulverförmiger Form im Reaktionsmedium dispergiert wird, gegebenenfalls ein Puffer gelöst oder in fester Form zugegeben wird und man, bevorzugt unter Rühren, unter den angegebenen Druck- und Temperaturbedingungen das Wasserstoffperoxid zulaufen läßt.

Beim erfindungsgemäßen Verfahren werden praktisch quantitative Ausbeuten und Selektivitäten von mehr als 98 % erzielt. Die erhältlichen Dithiazolyl-(2,2)-disulfide zeichnen sich durch hohe Reinheit aus, sie können ohne weitere Reinigung beispielsweise direkt als Gummivulkanisationsmittel eingesetzt werden.

Die erfindungsgemäß herstellbaren Dithiazolyl-(2,2')-disulfide eignen sich hervorragend als Vulkanisationsbeschleuniger in schwefelhaltigen Kautschukmischungen. Insbesondere geeignet ist das Dibenzothiazyldisulfid.

### Beispiele

### a)

In einem doppelwandigen, thermostatisierten 2 l Planschliffbecher, ausgestattet mit pH-Messelektrode mit angeschlossener Regeltechnik für zwei Dosierpumpen, Thermometer, Rührer, Tropftrichter und Stromstörer, wurde unter Inertgas (N₂) eine Suspension von 169 g (1 mol) 2-Mercaptobenzothiazol in 1 700 ml Wasser mit 500 ml Pufferlösung auf pH 7,18 eingestellt und unter Rühren auf 40°C erwärmt. Die Pufferlösung wurde hergestellt durch Auflösen von 15,14 g (12,5 mmol) Tris(hydroxymethyl)aminomethan in 250 ml Wasser, Zusatz von 210 ml 0,1 mol/l Salzsäure und Auffüllen der Lösung mit Wasser bis auf 500 ml. Innerhalb von 6 h wurden zu dem Reaktionsgemisch 600 ml einer verdünnten, wässrigen Wasserstoffperoxidlösung (0,525 mol H₂O₂) unter Rühren zugesetzt, wobei der pH durch eine Regeltechnik mit zwei Dosierpumpen im Bereich von 7 bis 7,5 gehalten wurde. Für diese Regelung wurden insgesamt 350 ml 1 %ige Natronlauge und 165 ml 2 %ige Schwefelsäure benötigt. Nach beendetem Zutropfen war der Test auf H₂O₂ (Iodstärkepapier) schwach positiv. Es wurde noch 30 min bei 40°C nachgerührt, filtriert und das Produkt mit Wasser gewaschen. Ausbeute: 163,1 g (98,1 % d. Th.), Wirkstoffgehalt nach Titration: 97,1 % MBTS, Fp.: 167-171°C.

### b)

In einem doppelwandigen, thermostatisierten 2 l Planschliffbecher, ausgestattet mit pH-Messelektrode mit angeschlossener Regeltechnik für zwei Dosierpumpen, Thermometer, Rührer, Tropftrichter und Stromstörer, wurde unter Inertgas (N₂) eine Suspension von 169 g (1 mol) 2-Mercaptobenzothiazol in 1 700 ml Wasser bei pH 6,80 unter Rühren auf 70°C erwärmt. Innerhalb von 6 h wurden 600 ml einer verdünnten, wässrigen Wasserstoffperoxidlösung (0,525 mol H₂O₂) unter Rühren zugesetzt, wobei der pH durch eine Regeltechnik mit zwei Dosierpumpen im Bereich von 7 bis 7,5 gehalten wurde. Für diese Regelung wurden insgesamt 230 ml 1 %ige Natronlauge und 140 ml 2 %ige Schwefelsäure benötigt. Nach beendetem Zutropfen war der Test auf H₂O₂ (Iodstärkepapier) schwach positiv. Es wurde noch 30 min bei 40°C nachgerührt, filtriert und das Produkt mit Wasser gewaschen. Ausbeute: 163,0 g (98,0 % d. Th.), Wirkstoffgehalt nach Titration: 98,8 % MBTS, Fp.: 169,5 bis 172,5°C.

### c) Vergleichsbeispiel

Mit der oben beschriebenen Versuchsanordnung und den nachfolgend angegebenen Bedingungen wurde der Versuch bei niedrigerem pH wiederholt. Folgende Parameter wurden eingestellt und während der Reaktion eingehalten: **pH 5,0 bis 5,5**, Temperatur 40°C, zur pH-Regelung wurden 170 ml 1 %ige Natronlauge verbraucht. Ausbeute: 162,2 g (97,5 % d.Th.), Wirkstoffgehalt: 69,7 % MBTS, Fp.: 142,0 bis 151,0°C.

### d) Vergleichsbeispiel

Mit der oben beschriebenen Versuchsanordnung und den nachfolgend angegebenen Bedingungen wurde der Versuch bei höheren pH wiederholt. Folgende Parameter wurden eingestellt und während der Reaktion eingehalten: **pH 8,1 bis 11,1**, Temperatur 40°C, zur pH-Regelung wurden 40 g 10 %ige Natronlauge verbraucht. Ausbeute: 150,6 g (90,6 % d.Th.), Wirkstoffgehalt: 88,5 % MBTS, Fp.: 166,0 bis 170,0°C.

## Patentansprüche

1. Verfahren zur Herstellung von Dithiazolyl-(2,2')-disulfiden der allgemeinen Formel 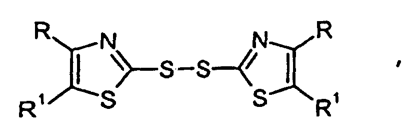 wobei
R und R¹ gleich oder verschieden sein können und jeweils für Wasserstoff, Halogen, Nitro, Hydroxyl oder gegebenenfalls substituiertes C₁-C₁₂-Alkyl oder -Alkoxyl oder C₆-C₁₂-Cycloalkyl oder -Aryl oder C₁-C₁₂-Heteroaryl stehen oder gemeinsam den Rest 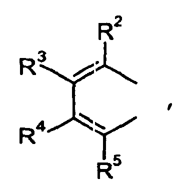
wobei
R² bis R⁵ gleich oder verschieden sein können und jeweils für Wasserstoff, Halogen, Nitro, Hydroxyl oder gegebenenfalls substituiertes C₁-C₁₂-Alkyl oder -Alkoxyl oder C₆-C₁₂-Cycloalkyl oder -Aryl oder C₁-C₁₂-Heteroaryl stehen,
bilden, durch Oxidation eines entsprechenden substituierten 2-Mercaptothiazols mit peroxidischen Verbindungen, **dadurch gekennzeichnet, daß** die Oxidation in wässriger Suspension bei einem pH-Wert im Bereich von 6,5 bis 8,0 durchgeführt wird.

2. Verfahren nach Anspruch 1, **dadurch gekennzeichnet, dass** man bei einem pH-Wert im Bereich von 6,8 bis 7,5 arbeitet.

3. Verfahren nach Anspruch 1, **dadurch gekennzeichnet, dass** man als peroxidische Verbindungen Wasserstoffperoxid, Alkylhydroperoxide und/oder Aralkylhydroperoxide einsetzt.

4. Verfahren nach Anspruch 1, **dadurch gekennzeichnet, dass** man Wasserstoffperoxid als Oxidationsmittel einsetzt.

5. Verfahren nach Anspruch 1, **dadurch gekennzeichnet, dass** man der wässrigen Suspension noch ein oxidationsstabiles, organisches Lösungsmittel zusetzt.

6. Verfahren nach Anspruch 1, **dadurch gekennzeichnet, dass** man als Ausgangsprodukt 2-Mercaptobenzothiazol einsetzt.

## Claims

1. Process for the production of 2,2'-dithiazolyl disulfides of the general formula 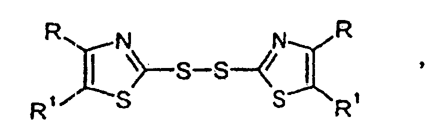 wherein
R and R¹ can be the same or different and each denote hydrogen, halogen, nitro, hydroxyl or optionally substituted C₁-C₁₂ alkyl or alkoxyl or C₆-C₁₂ cycloalkyl or aryl or C₁-C₁₂ heteroaryl, or jointly form the residue 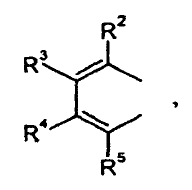
wherein
R² to R⁵ can be the same or different and each denote hydrogen, halogen, nitro, hydroxyl or optionally substituted C₁-C₁₂ alkyl or alkoxyl or C₆-C₁₂ cycloalkyl or aryl or C₁-C₁₂ heteroaryl,
by oxidation of a corresponding substituted 2-mercaptothiazole with peroxidic compounds, **characterised in that** the oxidation is performed in an aqueous suspension at a pH in the range of 6.5 to 8.0.

2. Process according to claim 1, **characterised in that** work is performed at a pH in the range of 6.8 to 7.5.

3. Process according to claim 1, **characterised in that** hydrogen peroxide, alkyl hydroperoxides and/or aralkyl hydroperoxides are used as the peroxidic compounds.

4. Process according to claim 1, **characterised in that** hydrogen peroxide is used as the oxidising agent.

5. Process according to claim 1, **characterised in that** an organic solvent that is stable to oxidation is also added to the aqueous suspension.

6. Process according to claim 1, **characterised in that** 2-mercaptothiazole is used as the starting product.

## Revendications

1. Procédé de préparation de dithiazolyl-(2,2')-disulfures de formule générale 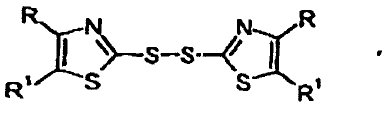 dans laquelle
R et R¹ peuvent être identiques ou différents et désignent respectivement l'hydrogène, un halogène, un radical nitro, hydroxyle ou un radical, éventuellement substitué, C₁-C₁₂-alkyle ou -alcoxyle ou C₆-C₁₆-cycloalkyle ou -aryle ou C₁-C₁₂-hétéroaryle ou, ensemble, forment le reste 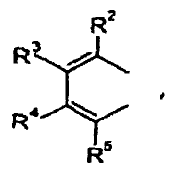 où
R² à R⁵ peuvent être identiques ou différents et désignent respectivement l'hydrogène, un halogène, un radical nitro, hydroxyle ou un radical, éventuellement substitué, C₁-C₁₂-alkyle ou -alcoxyle ou C₆-C₁₆-cycloalkyle ou -aryle ou C₁-C₁₂-hétéroaryle,
par oxydation d'un 2-mercaptothiazole substitué correspondant avec des composés peroxydiques, **caractérisé en ce que** l'oxydation est effectuée en suspension aqueuse à un pH de l'ordre de 6,5 à 8,0.

2. Procédé selon la revendication 1, **caractérisé en ce que** l'on travaille à un pH de l'ordre de 6,8 à 7,5.

3. Procédé selon la revendication 1, **caractérisé en ce que** l'on utilise comme composés peroxydiques du peroxyde d'hydrogène, des hydroperoxydes alkyliques et/ou des hydroperoxydes aralkyliques.

4. Procédé selon la revendication 1, **caractérisé en ce que** l'on utilise du peroxyde d'hydrogène comme oxydant.

5. Procédé selon la revendication 1, **caractérisé en ce que** l'on ajoute encore à la suspension aqueuse un solvant organique stable à l'oxydation.

6. Procédé selon la revendication 1, **caractérisé en ce que** l'on utilise du 2-mercaptobenzothiazole comme produit de départ.
